# EUROPEAN PATENT APPLICATION

(11) **EP 3 993 565 A1**
(43) Date of publication of application: **04.05.2022**
(21) Application number: 20205378.1
(22) Date of filing: 03.11.2020
(51) Int. Cl.: H05B 47/115, A61B 5/02, G01S 13/88, G01S 7/41

(54) **ENHANCED SIGNAL PROCESSING FOR A RADAR-BASED PRESENCE SENSOR**

(71) Applicant: Tridonic GmbH & Co. KG, 6851 Dornbirn (AT)
(72) Inventor: Kistler, Roger, 6850 Dornbirn (AT); Huang, Pengcheng, 6850 Dornbirn (AT); Künzli, Markus, 6850 Dornbirn (AT)
(74) Representative: Beder, Jens

(57) **Abstract**

An enhanced signal processing method for a radar-based presence sensor enables to control a building automation system using at least one sensor device. A sensor signal processing system for a building automation system comprises a signal processing circuit, which is configured to obtain a sensor signal acquired by a sensor device and to evaluate the sensor signal to detect characteristics indicating a heartbeat or respiration in the sensor signal. The signal processing circuit is configured to generate a detection signal comprising information on presence of at least one person based on the evaluated sensor signal, and to output the detection signal to the building automation system. The signal processing circuit is configured to determine different heartbeat rates or respiration rates in the sensor signal, and to determine a number of persons in the surveillance area based on the determined different heartbeat rates or respiration rates. The signal processing circuit maybe arranged locally in the sensor device, or alternatively in a remote server, which performs processing on one or more sensor signals acquired from corresponding sensor devices via a network.

## Description

The invention is in the field of control building automation systems, such as lighting systems, and sensors used for such systems. In particular, the invention concerns enhanced signal processing for RADAR-based presence sensors, and a sensor signal processing system.

Presence sensors provide valuable information for controlling lighting systems, air conditioning systems, or the like. Presence sensors may employ the radio detection and ranging (RADAR) measurement principle to detect the presence of persons in a surveillance area of the presence sensor.

Current presence sensors provide information on a detection of a person in a sensor surveillance area. If a count result on a number of present persons in the sensor surveillance area is required, a known approach performs a spatial evaluation of the received sensor signals for determining individual locations of the persons in the sensor surveillance area.

This approach fails in scenarios in which individual persons are at locations close to each other. The approach further suffers from high demands on spatial measurement resolution.

Intelligent presence sensors of current lighting systems use passive infrared (PIR) technology. These presence sensors show a high sensitivity so that they are able to detect human motion within a large surveillance area. However, they cannot detect successfully a person that is sitting in an office reading, typing, or watching a video, due to the lack of significant movements of the person with its arms or body. Another example of a failed detection by PIR sensors may include PIR sensors installed in a conference room where people are watching a screen without moving. Using PIR sensors in this application scenario may result in switching the luminaires off after some time elapsed because a PIR sensor does not detect any presence in the conference room and a controller of the lighting system reacts accordingly.

Alternative approaches for detecting the presence of humans, including the determination of the number of people, rely on image processing of signals acquired by cameras. These approaches benefit from an increased spatial resolution and thus improve count results on the number of present persons in the image area, but suffer from privacy concerns and the high complexity of image processing and corresponding high cost for sensors and sensor signal processing. Addressing the problem of determining the number of individuals present in a monitored area is therefore highly desirable.

In particular, the invention addresses the task of determining the number of persons present in a surveillance area of a presence sensor applying the RADAR principle.

The sensor signal processing system, the sensor device and the method for determining a number of present persons in a surveillance area provide advantageous solutions to the problem.

A sensor signal processing system for a building automation system comprises a signal processing circuit configured to obtain a sensor signal acquired by a sensor device. The signal processing circuit is further configured to evaluate the sensor signal to detect characteristics indicating a heartbeat or a respiration in the sensor signal, to generate a detection signal comprising information on presence of at least one person based on the detected characteristics, and to output the detection signal to the building automation system. The signal processing circuit is characterized in that the signal processing circuit is configured to determine different heartbeat rates or respiration rates in the sensor signal, and to determine a number of persons in the surveillance area based on the determined different heart beat rates or respiration rates.

The invention proposes using a current RADAR-based presence sensor (RADAR sensor) adapted to determine an individual heartbeat of a person for counting the number of persons in the surveillance area of the presence sensor. The generally known presence sensor is combined with a specific sensor signal processing, which detects characteristics in the sensor signal responsive to individual heartbeat rates of the persons, which are present in the sensor's surveillance area and discriminates between individual heartbeat rates (number of contractions of the heart -beats- per minute) and determines a count of individual heart rates from the sensor signal. The count of individual heartbeat rates in the sensor signal enables to discriminate between individual persons, and to conclude the count number of persons, which are present within the surveillance area of the presence sensor.

The method works equally for heartbeat rates as for respiration rates.

The proposed signal processing is advantageous, as the required amendment in the known RADAR sensor only includes an additional signal processing of the output signal. An increase in spatial sensor detection resolution in order to discriminate reliably between individual persons situated close together would require significant amendments in RADAR system design concerning hardware parameters, e.g. antenna parameters, RADAR signal generation parameters, e.g. waveform design, and sensor signal processing parameters, e.g. signal evaluation alike. The invention avoids these significant and costly amendments in RADAR sensor design and relies on an additional and specific sensor signal processing instead.

The sensor signal processing system according to an embodiment comprises the signal processing circuit configured to evaluate the sensor signal including determining a change of heartbeat rate or respiration rate from the sensor signal for individual persons. The signal processing circuit is configured to determine a physical condition of the at least one person based on the determined change of the heart beat rate or respiration rate of the at least one person.

Determining and evaluating an individual heartbeat rate of a present person and a change thereof enables deducing information on a physical condition of the corresponding person, and therefore determining if an emergency situation might apply in the current scenario.

The signal processing circuit of an advantageous embodiment is configured to evaluate the sensor signal including filtering the sensor signal for detecting the heartbeat or the respiration of the least one person.

In an embodiment of the sensor signal processing system, the signal processing circuit is configured to evaluate the sensor signal including evaluating a signal amplitude of the sensor signal. The signal processing circuit generates the detection signal based on the detected characteristics indicating the heartbeat rate or respiration rate of the least one person, and further based on the evaluated signal amplitude of the sensor signal.

An additional amplitude evaluation of the sensor signal may improve discrimination results for the combined evaluation of signal amplitude and heartbeat rate and respiration rate.

The sensor signal processing system according to an embodiment includes an actuator control circuit configured to generate a control signal based on the detection signal, the detection signal including the information on a number of persons in the surveillance area, and to output the control signal to at least one actuator of the building automation system.

The control signal thus takes into regard the number of persons in the surveillance area of the sensor. Alighting system may be controlled with improved precision using the additionally acquired data on the number of persons present, and possibly even considering their physical condition derived from the determined heartbeat rates or respiration rates. For example, a controller of the lighting system may increase light intensity in an area when becoming aware of a large number of persons being present in the area, and/or detecting unusually high heartbeat rates and/or increasing heartbeat rates of persons present in the surveillance area, possibly indicating an emergency.

Knowledge on the number of persons present in an area maybe advantageous for other applications than lighting control. For example, controlling an air conditioning system may benefit at least similarly from knowing the number of persons in a room. Adjusting the volume of the airflow of an air conditioning system can reasonably reduce the energy needed. Furthermore, infection disease control may require knowing the number of persons present in a room or even over a facility in order to control entry of persons into the room or facility so that under any circumstances the number of present persons per area remains below a predetermined threshold.

The sensor signal processing system according to an embodiment shows the signal processing circuit arranged in the sensor device.

Preferably, the sensor signal processing is performed in a sensor device (presence sensor device) using at least one digital signal processor, and the sensor device provides the detection signal including detection data (persons are detected) and count data (number of persons determined) at a signal output of the sensor device. Performing local sensor signal processing in the sensor device offers advantages with respect to data privacy and data protection issues. The sensor device provides only anonymous data on persons at the output of the sensor device without enabling conclusions on the provided data who is actually present in the sensor surveillance area. Bandwidth requirements for the detection signal are advantageously small due to transmitting only processed detection data and count data instead of measurement signals for real-time processing to a remote location.

The sensor signal processing system according to an alternate embodiment has the signal processing circuit configured to obtain the output signal acquired by the sensor device arranged separate and remote from the signal processing circuit.

This embodiment enables an economic use of signal processing resources for sensor signals received from a plurality of sensor devices, for example, a facility with a large number of rooms.

The sensor signal processing system according to an embodiment further comprises a communication interface configured to receive a communication signal from the at least one sensor device, wherein the communication signal includes the output signal of the at least one sensor device and identification information of the at least one sensor device. The communication interface is configured to extract the output signal from the communication signal, and to provide the output signal to the signal processing circuit.

In case the sensor signal is provided to a central signal processing facility for evaluation, arranged at a remote location to the location of the sensor, a better utilization of specialized and complex signal processing hardware for the evaluation of the sensor signal is achieved, as the central signal processing facility may combine processing of sensor signals of a plurality of sensors, each sensor being unambiguously identifiable.

The sensor signal processing system may further comprise an actuator control circuit configured to obtain the detection signal and to generate a control signal for controlling at least one actuator of the building automation system based on the detection signal, and to output the control signal to the at least one actuator.

A sensor device for a building automation system comprises the sensor configured to provide the output signal, and the signal processing system.

In preferred embodiment, the sensor of the sensor device is a RADAR sensor, in particular a CW RADAR sensor or a FMCW RADAR sensor.

FMCW RADAR sensors for presence detection are already available and offer the basic measurement capabilities for heart activity measurements, for example heartbeat rate (HR) and heart rate variability (HVR) signal measurements and respiration activity measurements.

The building automation system may be a lighting system, in particular an emergency lighting system.

The enhanced signal processing represents a commercially advantageous additional function for emergency lighting systems already required in many public areas.

A method for determining the number of persons present in a surveillance area using at least one sensor device. The method comprises a step of obtaining, by a signal processing circuit, a sensor signal from the at least one sensor device. The method proceeds by the signal processing circuit evaluating the acquired sensor signal for detecting characteristics indicating a heartbeat or a respiration in the acquired output signal. The method generates, by the signal processing circuit, a detection signal comprising information on presence of at least one person. The method is characterized in that evaluating the sensor signal by the signal processing circuit, includes determining different heartbeat rates or respiration rates in the sensor signal, and determining a number of persons in the surveillance area based on the determined different heart beat rates or respiration rates.

Embodiments are discussed with reference to the attached figures, in which
- Fig. 1: shows an application scenario for determining the number of persons in a surveillance area of a radar based presence sensor,
- Fig. 2: depicts characteristic signals when determining the number of persons in a surveillance area of a radar based presence sensor in a scenario corresponding to fig. 2,
- Fig. 3: provides a block-diagram of a sensor device applying an enhanced signal processing for determining the number of persons in a surveillance area of a RADAR- based presence sensor,
- Fig. 4: provides a block-diagram of an alternate embodiment for applying the enhanced signal processing for determining the number of persons in a surveillance area of a RADAR-based presence sensor, and
- Fig. 5: provides a simplified flowchart for a method for determining the number of persons in a surveillance area of a radar based presence sensor.

In the figures, same reference signs denote same or corresponding elements. The discussion of the figures avoids discussing same or corresponding elements with same reference signs in different figures for sake of conciseness.

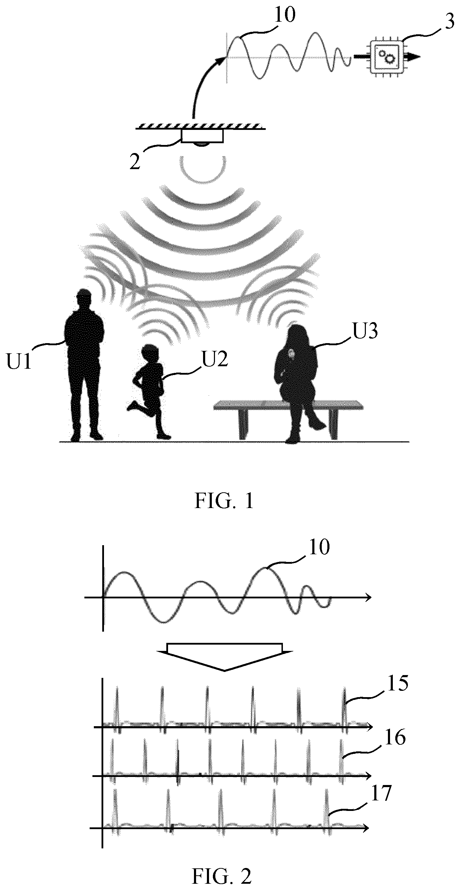

Fig. 1 depicts an application scenario for determining the number of persons in a surveillance area of a RADAR-based presence sensor 2.

The presence sensor 2 may include a RADAR sensor and may be mounted at the ceiling of a room in a building. The presence sensor 2 uses at least one antenna to emit a RADAR signal into at least a portion the room defined by an antenna coverage diagram of the antenna and to receive a receive signal 6 reflected from objects located in the portion of the room.

The antenna coverage diagram defines the surveillance area of the RADAR sensor. The surveillance area corresponds to a footprint of an antenna diagram of the antenna on the floor of the room. The antenna diagram determines the surveillance area of the RADAR sensor.

The use of multiple antennas, and/or separate transmit antennas and receive antennas is generally known.

The receive signal 6 includes in particular receive signal components from the radar signal reflected by persons U1, U2, U3 located in the surveillance area of the presence sensor 2.

The presence sensor 2 processes the receive signal 6 and generates a sensor signal 10 based on the receive signal 6. The sensor signal 10 comprises in particular characteristics, which each are indicative of an individual heartbeat rate or an individual perspiration rate of the persons U1, U2, U3 in the surveillance area.

The characteristics may include phase variations obtained by an evaluation of the receive signal 6 and the RADAR signal 5.

The invention uses the sensor signal 10 as an input signal to a signal processor circuit 3. The signal processor circuit 3 performs an evaluation of the sensor signal 10. The sensor signal 10 may be a pre-processed sensor signal 10 provided by the presence sensor 2. The sensor signal 10 can be a pre-processed baseband signal at the output of a RADAR transceiver of the presence sensor 2.

Fig. 2 depicts characteristic signal components 15, 16, 17 when determining the number of persons U1, U2, U3 in the surveillance area of the RADAR-based presence sensor 2 in a scenario corresponding to fig. 1.

The signal processor circuit 3 performs an evaluation of the sensor signal 10, which includes filtering of the sensor signal 10 in order to determine individual heart-beat rates or respiration rates in the sensor signal 10. The determined number of individual heartbeat rates or respiration rates in the sensor signal 10 are shown in fig. 2 as three characteristic signal component 15, 16, 17, each corresponding to one of the three persons U1, U2, U3 situated in the surveillance area of the presence sensor 2.

An object in the surveillance area with a quasiperiodic movement reflects the emitted RADAR signal with its phase modulated by the time-varying position of the object according to the Doppler effect. When the object includes a chest of persons U1, U2, U3, the reflected RADAR signal contains characteristic information indicative of a chest displacement, which occurs due to heartbeat and/or respiration of the person U1, U2, U3. While holding breath, however, the reflected RADAR signal depends on a varying signal runtime due to varying chest displacement of the person U1, U2, U3 due to heartbeat alone. Typical values of the variation of the chest displacement of a person U1, U2, U3 at rest, caused by respiration, is between 4 and 12 mm, and the chest displacement due to heartbeat alone ranges between 0.2 and 0.5 mm. The respiration rate corresponds to a frequency that varies between 0.1 and 0.3 Hz, while the heartbeat rate (HR) corresponds to a frequency that varies between 1 and 3 Hz.

The inventive signal processing uses known methods to detect characteristics indicative of heartbeat rates or respiration rates in the sensor signal 10 by an evaluation of the sensor signal 10. The signal processing circuit 3 discriminates between different individual heartbeat rates or respiration rates in the sensor signal 10 based on the detected characteristics in the sensor signal 10. The signal processing circuit 3 determines different individual heartbeat rates or respiration rates in the sensor signal 10 based on the detected characteristics in the sensor signal 10. The signal processing circuit 3 determines a number of persons U1, U2, U3 in the surveillance area of the presence sensor 2 by determining the number of determined individual heartbeat rates or respiration rates in the sensor signal 10 based on the detected characteristics in the sensor signal 10.

Fig. 2 shows three characteristic signal component 15, 16, 17, each corresponding to one of the three persons U1, U2, U3 situated in the surveillance area of the presence sensor 2. Each characteristic signal component 15, 16, 17, may have a particular frequency corresponding to a particular heartbeat rate or respiration rate, a particular signal phase and particular signal amplitude characteristics.

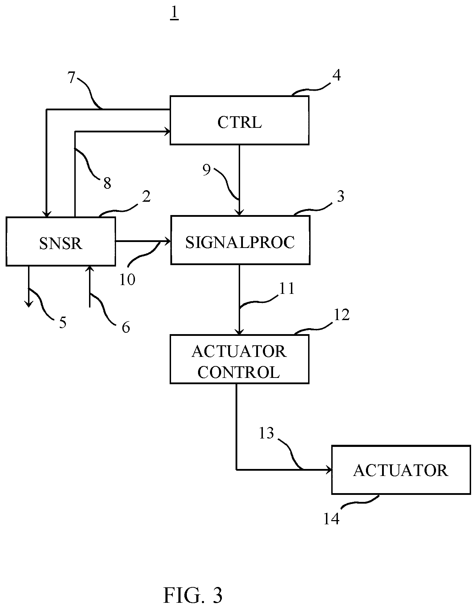

Fig. 3 shows a sensor device applying an enhanced signal processing for determining the number of persons U in the surveillance area of the presence sensor 2 in a simplified block-diagram.

The presence sensor 2 applies the FMCW-RADAR principle. The frequency-modulated, continuous wave radio detection and ranging (FMCW-RDAR)-technology is generally known for application in the presence sensor 2.

The transmit frequency (carrier frequency) of the RADAR signal may be in the 24 GHz frequency band reserved for industrial, scientific and medical (ISM) applications other than telecommunications. The transmit frequency maybe in other frequency bands,for example the radar sensor may operate on a transmit frequency of 1.6 GHz or 2.4 GHz, 5.8 GHz or 16 GHz also in use for Doppler RADAR systems for heartbeat rate and hear rate variability signal detection. In particular, the frequency of 5.8 GHz is commonly used as operation frequency.

The presence sensor 2 (radar sensor) emits a RADAR signal 5 (radio signal) via an antenna. The presence sensor 2 illuminates the surveillance area with the RADAR signal. The antenna emits the wireless RADAR signal 5 (radio signal) as an electromagnetic wave into the surveillance area. The antenna has a characteristic antenna diagram, which defines the extension of the surveillance area of the presence sensor 2. Objects in the surveillance area reflect the RADAR signal 5.

The presence sensor 2 may have a monostatic radar sensor configuration or a bi-static radar sensor configuration. The radar sensor may use one transmit antenna, and one or multiple receive antennas in the bi-static radar sensor configuration.

The presence sensor 2 emits the RADAR signal 5 with a specific signal waveform (radar signal waveform). The signal waveform may include a saw-tooth frequency modulation, triangle frequency modulation, rectangular frequency modulation, e.g. frequency shift keying (FSK), stepwise frequency modulation or sinusoidal frequency modulation.

A time dependent variation of the carrier frequency by a frequency modulation (FM) enable performing signal runtime measurements using the presence sensor 2.

A modulation maybe turned off and on in alternate signal periods to enable determining object velocity using unmodulated transmit frequency shift based on a Doppler shift included in the receive signal 6.

Each object has a characteristic RADAR cross section (RCS), which describes how detectable an object is by a radar sensor. The RCS defines the electromagnetic signature of the object. The RCS depends on the material of which the target is made, the size of the object relative to the wavelength of the illuminating radar signal 5, an absolute size of the object, and an incident angle. The incident refers to an angle at which the radar signal 5 (radar beam) hits a particular portion of the object, which depends upon the shape of the object and its orientation to the radar sensor. Furthermore, the RCS depends on the reflected angle at which the reflected radar signal leaves the part of the object illuminated by the radar sensor, the reflected angle depends upon incident angle. Furthermore, the RCS depends on the polarization of the transmitted radio signal and the reflected radio signal with respect to the orientation of the object.

If the reflecting object is moving with a velocity component radially to the radar sensor, the reflected radar signal is shifted by a Doppler frequency f_{D} with respect to the transmit frequency of the radar signal.

Individual objects in the surveillance area are detected using the Doppler-effect, which causes the reflected RADAR signal to have a different frequency than the emitted RADAR signal 5.

The presence sensor 2 receives the reflected RADAR signal 5 as receive signal 6. The receive signal 10 is processed to generate the sensor signal 10. The presence sensor 2 outputs the sensor signal 10 to the signal processing circuit 3 for further processing.

By applying frequency filtering on the receive signal 6, the radar sensor detects objects in the surveillance area suppressing the frequency of the emitted radar signal 5. The presence sensor 2 may detect small movements of a human body, such as breathing, speaking or typing. The radar sensor acquires and provides information on a location, where a movement in the surveillance area occurs, measured as a distance to the presence sensor 2.

The signal processing system 1 may include a control circuit 4. The control circuit 4 generates a control signal 7 for controlling the presence sensor 2 and a further control signal 7 for controlling the signal processing circuit 3. The control signal 7 and the further control signal 9 may include clock signals for controlling the operation of the presence sensor 2 and the signal processing circuit 3. This enables a coherent signal processing and evaluation of the sensor signal 10 by the signal processing circuit 3.

The control circuit 4 may also receive status signals from the presence sensor 2 and the signal processing circuit 3. For example, the presence sensor 2 may provide a sensor status signal 8 to the control circuit 4.

Electronic circuitry forming part of the presence sensor 2 and/or the signal processing circuit 3 may implement the control circuit 4.

The signal processing circuit 3 may be a structural element included in a sensor device also comprising the presence sensor 2. In the embodiment of fig. 3, the signal processing of the sensor signal 10 is performed locally by the signal processing circuit 3 co-located with the presence sensor 2.

The signal processing circuit 3 may include at least one microcontroller or digital signal processor.

The signal processing circuit 3 performs signal processing of the obtained sensor signal 10.

The signal processing circuit 3 evaluates the obtained sensor signal 10 to detect characteristics in the sensor signal 10 indicating a heartbeat or respiration. In particular, signal processing circuit 3 evaluates the obtained sensor signal 10 to detect characteristics in the sensor signal 10 indicating individual heartbeat rates or respiration rates. The signal processing circuit 3 determines different heartbeat rates or respiration rates in the sensor signal 10 based on the characteristics in the sensor signal 10. The signal processing circuit 3 determines a number of persons U in a surveillance area of the sensor device 2 based on the determined different heartbeat rates or respiration rates.

The signal processing circuit 3 generates a detection signal 11 based on the evaluation of the sensor signal 10. The detection signal 11 comprises information on the number of detected individual heartbeat rates or a respiration rates present in the receive signal 10. Thus, the detection signal 11 comprises information on the number of persons U in the surveillance area based on the evaluated sensor signal 10 obtained from the presence sensor 2.

The signal processing circuit 3 may perform detecting heartbeat rates or respiration rates of persons U using generally known algorithms for Doppler RADAR systems used to detect heartbeat signals for monitoring purposes of patients replacing traditional electrocardiograms.

The signal processing circuit 3 may evaluate the sensor signal 10 including filtering the receive signal 10 for detecting the heartbeat rate or the respiration rate of the least one person U.

The signal processing circuit 3 may perform signal processing on the receive signal 10 to evaluate the sensor signal 10 including measuring signal amplitudes of the receive signal 10.

The signal processing circuit 3 generates the detection signal 11 based on the detected heartbeat rates or respiration rates of persons U, and in particular including data on the number of the detected heartbeat rates or respiration rates in the evaluated receive signal 10. The signal processing circuit 3 may generate the detection signal 11 further based on the measured signal amplitudes of the receive signal 10, in particular amplitudes of the signal components 15, 16, 17, in the sensor signal 10 corresponding to the individual detected heartbeat rates or respiration rates detected in the receive signal 10.

The signal processing circuit 3 may determine a change, in particular a measure for a change, for each determined individual heartbeat rate or respiration rate in the sensor signal 10 for the individual person U1, U2, U3. The signal processing circuit 3 may then determine a physical condition of the at least one person U1, U2, U3 based on the determined change of the heartbeat rate or respiration rate of the at least one person U1, U2, U3.

The signal processing circuit 3 outputs the generated detection signal 11 to the building automation system.

The detection signal 11 includes information on the number (count) of persons U, whose presence the signal processing circuit 3 detected in the sensor signal 10 obtained from the presence sensor 2.

The detection signal 11 may comprise information on determined heartbeat rates or respiration rates in the receive signal 10. For example, the detection signal 11 may include data on an average heartbeat rate or an average respiration rate calculated by the signal processing circuit 3 when evaluating the obtained sensor signal 10. The signal processing circuit 3 may compute the average heartbeat rate or an average respiration rate over a predefined period of time (time window) for each detected individual heartbeat rate or respiration rate. Additionally or alternatively, the signal processing circuit 3 may compute the average heartbeat rate or an average respiration rate over all detected individual heart beat rates or respiration rates in the sensor signal 10.

The detection signal 11 may comprise information on a determined change of heartbeat rates or changes of respiration rates in the sensor signal 10 for each individual person U1, U2, U3 or over all determined persons U1, U2, U3 in the surveillance area.

The detection signal 11 may include information on a physical condition of each individual person U based on the determined rate of change of the heartbeat rate or respiration rate of the person U in the surveillance area. Additionally or alternatively, the detection signal 11 may include information on the combined physical condition of all individual persons U1, U2, U3 based on the determined change of the heartbeat rates or respiration rates of the persons U1, U2, U3 in the surveillance area.

The signal processing circuit 3 provides the detection signal 11 to the building automation system, in particular to an actuator control processor 12 of the building automation system. The actuator control processor 12 generates a control signal 13 based on the detection signal 11 and provides the generated control signal 13 to at least one actuator 14 for controlling the function performed by the actuator 14.

The actuator control processor 12 may extract a current number of persons U1, U2, U3 present in the surveillance area of each corresponding presence sensor 2. The actuator control processor 12 may then compare the extracted current number of persons U1, U2, U3 with a threshold value and generate a control signal based on a result of the comparison.

For a lighting system as one example for the building automation system, the generated control signal 13 may indicate that the number of persons U1, U2, U3 in the surveillance area exceeds the threshold and a light intensity of at least one luminaire arranged in the surveillance area may be increased to avoid a panic in the surveillance area.

For an air conditioning system as another example for the building automation system, the generated control signal 13 may indicate that the number of persons U1, U2, U3 in the surveillance area exceeds the threshold and therefore increase an airflow of an air conditioner arranged in the surveillance area to ensure a proper air conditioning of a room.

The actuator control processor 12 provides the generated control signal 13 to the at least one actuator 14 for controlling operation of the actuator 14. The actuator may be any kind of actuator 14 performing a function in a building automation system. The at least one actuator 14 may, for example, include a luminaire, an air conditioning unit, a drive motor for a window shutter, a drive motor for opening/closing a window, a traffic light controlling entrance to a building, an emergency exit sign, a loudspeaker of a public address system, or a distress call unit.

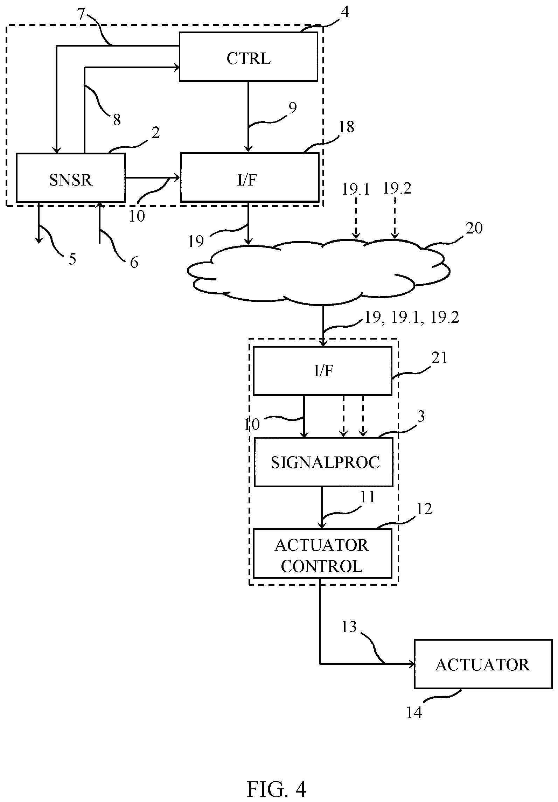

Fig. 4 shows a block-diagram of an alternate embodiment for applying the enhanced signal processing for determining the number of persons in a surveillance area of a RADAR-based presence sensor 2.

The presence sensor 2 performs a pre-processing to generate the sensor signal 10 as discussed in the embodiment shown in fig. 3. In the alternate embodiment of fig. 4, the presence sensor 2 provides the sensor signal 10 to a sensor communication interface 18. The sensor communication interface 18 generates a communication signal 19, which comprises the sensor signal 10 and identification data identifying the presence sensor 2. The generated communication signal 19 may include further information and data, for example status data of the presence sensor 2.

The sensor communication interface 18 provides the communication signal 19 via a network 20 (communication network) to a communication interface 21 of a remote server. The remote server comprises the signal processing circuit 3, which performs signal processing, in particular evaluation of the sensor signal 10 in a corresponding manner to the embodiment discussed with reference to fig. 3.

The remote server may process sensor signals 10 obtained from a plurality of presence sensors 2 via the network 20 in corresponding communication signals 19, 19.1, 19.2.

The remote server may include the communication interface 21 configured to receive the communication signal 19 from the at least one presence sensor 2, wherein the communication signal includes the sensor signal 10 from the at least one presence sensor 2. The communication interface 21 processes the received communication signal 19, extracts the receive signal 10 from the communication signal 19 and provides the extracted sensor signal 10 associated with identification information on the corresponding presence sensor 2 from which the sensor signal 10 stems to the control circuit 3 for further processing.

The remote server may further include the actuator control circuit 12 for obtaining the detection signal 11 generated by the signal processing circuit 3. The actuator control circuit 12 generates the control signal 14 for controlling at least one actuator 14 of the building automation system based on the detection signal 11.

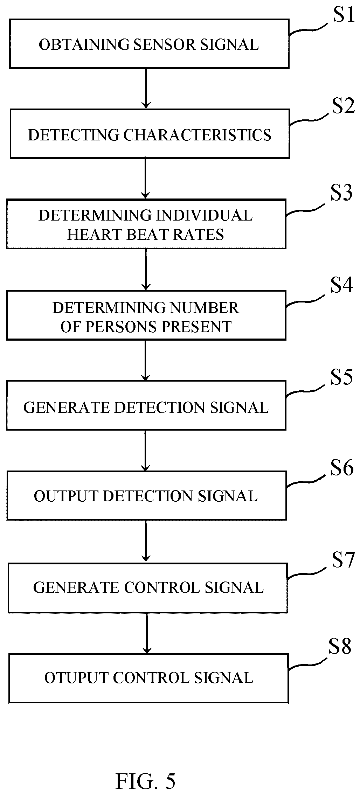

The at least one actuator 14 may output the control signal 13 to the at least one actuator 14 directly. Alternatively or additionally, the at least one actuator 14 may output the control signal 13 to the at least one actuator 14 via the communication interface
Fig. 5 provides a simplified flowchart for a method for determining the number of persons U1, U2, U3 in the surveillance area of the RADAR-based presence sensor 2.

The method starts with the signal processing circuit 3 obtaining a sensor signal 10 from a sensor device, which includes the presence sensor 2 in step S1.

The signal processing circuit 3 proceeds by evaluating the obtained receive signal 10 to detect heartbeat rates or respiration rates in the sensor signal 10 in steps S2 and S3.

In step S2, the signal processing circuit 10 evaluates the sensor signal 10 to detect characteristics in the sensor signal 10 indicating a heartbeat or respiration.

In step S3, the signal processing circuit 3 determines different individual heartbeat rates or respiration rates in the sensor signal 10 based on the detected characteristics.

Subsequently, the signal processing circuit 3 determines a number of persons U1, U2, U3 present in the surveillance area based on the determined different individual heartbeat rates or respiration rates in step S4.

In step S5, the signal processing circuit 3 generates a detection signal 11 based on the evaluated sensor signal 10. The detection signal 11 includes at least the information on a number of persons U1, U2, U3 which are present in the surveillance area monitored by the presence sensor 2.

The signal processing circuit 3 outputs the detection signal 11 to the actuator control circuit 12 in step S6.

In step S7, the actuator control circuit 12 generates a control signal 13 based on the detection signal 11, wherein the detection signal 11 includes at least the information on the number of persons U1, U2, U3 in the surveillance area.

The actuator control circuit 12 outputs the generated control signal 13 to at least one actuator 14 of the building automation system in step S8.

## Claims

1. Sensor signal processing system for a building automation system, the sensor signal processing system comprising
a signal processing circuit (3) configured to
obtain a sensor signal (10) output by a sensor device (2),
evaluate the sensor signal (10) to detect characteristics in the sensor signal (10) indicating a heartbeat or respiration ,
to generate a detection signal (11) comprising information on presence of at least one person (U) based on the detected characteristics,
to output the detection signal (11) to the building automation system, and **ch**
**aracterized in**
that the signal processing circuit (3) is configured to determine different heartbeat rates or respiration rates in the sensor signal (10), and to determine a number of persons (U) in a surveillance area of the sensor device (2) based on the determined different heartbeat rates or respiration rates.

2. The sensor signal processing system according to claim 1, wherein
the signal processing circuit (3) is configured to evaluate the sensor signal (10) including
determining a change of heartbeat rates or respiration rates in the sensor signal (10) for individual persons (U), and
determining a physical condition of the at least one individual person (U) based on the determined change of the heartbeat rate or respiration rate of the at least one individual person (U).

3. The sensor signal processing system according to any of the preceding claims, wherein
the signal processing circuit (3) is configured to evaluate the sensor signal (10) including filtering the sensor signal (10) for detecting the characteristics indicating heartbeat or the respiration of the least one person (U).

4. The sensor signal processing system according to any of the preceding claims, wherein
the signal processing circuit (3) is configured to evaluate the sensor signal (10) including evaluating a signal amplitude of the sensor signal (10), and generating the detection signal (11) based on the detected characteristics indicating heartbeat or respiration of the least one person (U), and further based on the evaluated signal amplitude of the sensor signal (10).

5. The sensor signal processing system according to any of the preceding claims, wherein
the signal processing circuit (3) is arranged in the sensor device (2).

6. The sensor signal processing system according to any of the preceding claims, wherein
the signal processing circuit (3) is configured to obtain the sensor signal (10) acquired by the sensor device (2) arranged separate and remote from the signal processing circuit (3).

7. The sensor signal processing system according to any of the preceding claims, wherein the sensor signal processing system further comprises
an interface circuit configured to receive a communication signal (19) from the at least one sensor device (2), wherein the communication signal (19) includes the sensor signal (10) of the at least one sensor device (2) and identification information of the at least one sensor device (2), to extract the sensor signal (10) from the communication signal (19), and to provide the sensor signal (10) to the signal processing circuit (3).

8. The sensor signal processing system according to any of the preceding claims, wherein the sensor signal processing system further comprises
an actuator control circuit (12) configured to obtain the detection signal (11) and to generate a control signal (14) for controlling at least one actuator (14) of the building automation system based on the detection signal (11), and to output the control signal (13) to the at least one actuator (14).

9. Sensor device for a building automation system, the sensor device comprising
the sensor configured to provide the sensor signal (10), and
a signal processing system (1) according to one of claims 1 to 8.

10. The sensor device for a building automation system according to claim 9, wherein
the sensor is a radar sensor, in particular a CW radar sensor or a FMCW radar sensor.

11. Method for determining the number of persons present in a surveillance area using at least one sensor device (2), the method comprising
obtaining (S1), by a signal processing circuit (3), a sensor signal from a sensor device (2), a sensor signal (10),
evaluating (S2), by the signal processing circuit (3), the sensor signal (10) to detect characteristics indicating a heartbeat or respiration in the sensor signal (10),
generating (S5) a detection signal (11) comprising information on presence of at least one person (U), and
**characterized in**
**that** evaluating the sensor signal (10) includes determining different heartbeat rates or respiration rates in the sensor signal (10) (S3), and determining a number of persons (U) in the surveillance area based on the determined different heartbeat rates or respiration rates (S4).

12. The method according to claim 11, wherein the method comprises a step of
outputting the detection signal (11) to a building automation system, in particular to a lighting system or an emergency lighting system.
